# EUROPÄISCHE PATENTSCHRIFT

(11) **EP 2 191 801 B1**
(45) Veröffentlichungstag und Bekanntmachung des Hinweises auf die Patenterteilung: **14.06.2017**
(21) Anmeldenummer: 09014556.6
(22) Anmeldetag: 23.11.2009
(51) Int. Cl.: A61F 13/62

(54) **Verbundstoffelement für einen Klettverschluss**
Compound material element for a hook-and-loop fastener
Elément composite pour une fermeture à crochet

(30) Priorität: 28.11.2008 DE 102008059512
(43) Veröffentlichungstag der Anmeldung: 02.06.2010
(73) Patentinhaber: Mondi Gronau GmbH, 48599 Gronau (DE)
(72) Erfinder: Baldauf, Georg, 48366 Laer (DE); Homölle, Dieter, 48607 Ochtrup (DE)
(74) Vertreter: Lorenz, Bernd Ingo Thaddeus

(56) Entgegenhaltungen:
- EP-A1- 1 302 582
- EP-A2- 2 103 293
- DE-U1- 9 321 556
- FR-A1- 2 794 348

## Beschreibung

Die Erfindung betrifft ein Verbundstoffelement für einen Klettverschluss, insbesondere einen Windelverschluss, mit einem Träger und einer auf den Träger aufkaschierten textilen Deckschicht, die freie Schlaufen für den Eingriff von Hakenelementen aufweist, wobei die Deckschicht aus einem Vliesstoff und daran eingewirkten polymeren Fäden, welche die Schlaufen bilden, besteht.

Das Verbundstoffelement bildet den weiblichen Teil eines Klettverschlusses. Bei der Verwendung an Windeln wird das Verbundstoffelement auf dem vorderen Bündchenbereich der Windel angebracht. Ein Verschlussband, das seitlich an der Windel befestigt ist und an seinem freien Ende Kletthaken aufweist, vervollständigt den Klettverschluss. Klettverschlüsse können mehrfach geöffnet und verschlossen werden, ohne dass dadurch die Funktionalität des Verschlusses leidet. Im Gegensatz zu Klebeverschlüssen sind Klettverschlüsse unempfindlich gegenüber Kontakt mit Hautcremes oder Puder.

An ein Verbundstoffelement für einen Klettverschluss an einem Wegwerfprodukt, z. B. Babywindeln, werden mehrere Anforderungen gestellt. Das Material soll ein möglichst geringes Flächengewicht aufweisen, damit es kostengünstig gefertigt werden kann. Trotz seines geringen Flächengewichtes muss das Material eine ausreichende Verhakung mit Kletthaken des zugeordneten Verschlussbandes gewährleisten. Erforderlich ist eine ausreichende Zahl von frei beweglichen Schlaufen, deren Funktion durch eine Verklebung des Trägers mit der textilen Deckschicht nicht beeinträchtigt werden darf. Nicht zuletzt soll das Material eine den Verbraucher ansprechende Anmutung besitzen.

Ein Verbundstoffelement mit den eingangs beschriebenen Merkmalen ist aus der Druckschrift EP 1 156 767 B1 bekannt, wobei als textile Deckschicht ein mehrlagiger Vliesstoff-Verbund vorgesehen ist und wobei texturierte polymere Fäden zur Bildung der Schlaufen in den gesamten Vliesstoff-Verbund eingewirkt sind. Um eine gute Druck- bzw. Klebefläche zu erreichen, kann die Deckschicht optional auf eine Folie als Träger aufkaschiert werden, auf welche dann der Druck bzw. die Klebeschicht aufgebracht wird. Sofern gemäß einer alternativen Ausgestaltung kein Träger vorgesehen ist, ergibt sich der Nachteil, dass der Vliesstoff-Verbund eine vergleichsweise große Dicke aufweisen muss, um eine ausreichende Festigkeit zu gewährleisten. Wenn der Vlies-Verbundstoff mit den eingewirkten polymeren Fäden ohne eine Folie als Träger verwendet werden soll, kann der Vliesstoff-Verbund an einer Außenlage mit einem Haftklebstoff versehen werden, um die Befestigung des Verbundstoffelementes, beispielsweise an einer Windel, zu ermöglichen. Die gegenüberliegende Seite kann dabei mit einem Aufdruck versehen werden. Nachteilig ist, dass der dann nach der Befestigung mittels des Haftklebstoffes außen liegende Aufdruck leicht abgerieben werden kann. Insbesondere kann es bei der Benutzung des Verbundstoffelementes zu unerwünschten Verschmutzungen kommen. Des Weiteren ist aufgrund der aus dem Vliesstoff-Verbund vorstehenden Schlaufen einerseits und der aus Kostengründen üblicherweise angestrebten niedrigen Flächengewichte andererseits ein gleichmäßiger Aufdruck nicht oder nur schwer möglich.

Die DE 10 2006 028 377 A1 beschreibt einen textilen Verbundstoff mit einem Träger und einer Deckschicht, die durch Nähfäden miteinander vernäht sind. Die Nähfäden bilden auf der Oberseite des Verbundstoffes Schlaufen für den Eingriff von Hakenelementen und auf der Unterseite des Verbundstoffes Maschen. Der Träger und die Deckschicht können aus Vliesstoffen bestehen. Hinsichtlich der Kletteigenschaften und der Verbundfestigkeit müssen Kompromisse eingegangen werden, da in einem Wirkprozess mit denselben Nähfäden sowohl freie Schlaufen erzeugt als auch die Schichten miteinander verbunden werden.

Die FR 2 794 348 A1 offenbart ein Verbundstoffelement für einen Klettverschluss, insbesondere einen Windelverschluss, mit einem Träger und einer auf dem Träger aufkaschierten textilen Deckschicht, die freie Schlaufen für den Eingriff von Hakenelementen aufweist, wobei die Deckschicht aus einem Vliesstoff und darin eingewirkten polymeren Fäden, welche die Schlaufen bilden, besteht. Die Schlaufen sind lediglich in den Vliesstoff der Deckschicht eingearbeitet. Die Deckschicht ist mit dem Träger verklebt.

Der Erfindung liegt die Aufgabe zugrunde, ein Verbundstoffelement anzugeben, welches sich durch eine angenehme Haptik und hochwertige visuelle Eigenschaften auszeichnet und gleichzeitig gute Gebrauchseigenschaften aufweist. Insbesondere soll das Verbundstoffelement mit einem abriebfesten Aufdruck bedruckt sein.

Ausgehend von einem Verbundstoffelement mit den eingangs beschriebenen Merkmalen wird die Aufgabe erfindungsgemäß dadurch gelöst, dass der Träger aus einem Nonwoven besteht, dass die Schlaufen lediglich in den Vliesstoff der Deckschicht eingearbeitet sind und dass die Deckschicht mit dem Träger verklebt ist.

Die Deckschicht des erfindungsgemäßen Verbundstoffelementes ist aus einem Vliesstoff und darin eingewirkten polymeren Fäden gebildet. Der Vliesstoff bildet ein flächiges Grundgerüst. Die polymeren Fäden sind in den Vliesstoff eingewirkt, d. h. in einem Wirkverfahren unter Bildung von Maschen eingearbeitet, und bilden freie Schlaufen für den Eingriff in Hakenelementen. Durch das Einwirken separater polymerer Fäden können in einer gleichmäßigen Anordnung Schlaufen genau definierter Größe erzeugt werden, welche eine besonders zuverlässige Verbindung mit Kletthaken eines Hakenelementes ermöglichen. Der Träger stellt eine von der Deckschicht unabhängige Lage dar, der dem Verbundstoff die Festigkeit und die für die Benutzung als Klettelement erforderliche Stabilität gibt. Der textile Träger einerseits und die Deckschicht mit darin eingewirkten Schlaufen andererseits können getrennt voneinander gefertigt und nachträglich durch eine Klebekaschierung verbunden werden. Das erfindungsgemäße Verbundstoffelement weist eine luftdurchlässige Struktur auf und zeichnet sich des Weiteren durch eine angenehme, weiche Haptik aus. Es erweckt einen sehr hochwertigen, textilen Eindruck.

Erfindungsgemäß ist der Träger auf der zur Deckschicht zugewandten innenliegenden Seite bedruckt. Der Aufdruck ist dabei in besonders vorteilhafter Weise durch die Deckschicht geschützt. Da die Deckschicht jedoch üblicherweise dünn und durchscheinend ausgeführt ist, bleibt der Aufdruck gut sichtbar. Aufgrund der typischerweise vorgesehenen Flächengewichte ist üblicherweise eine Bedruckung der Deckschicht nicht oder nur schwer möglich, während der Träger, der insbesondere eine ausreichende Formstabilität gewährleisten soll, vergleichsweise leicht bedruckt werden kann.

Zum Bedrucken des Trägers kann insbesondere ein Rotationsdruckverfahren vorgesehen sein. Das Rotationsdruckverfahren zeichnet sich trotz vergleichsweise hoher Einrichtungskosten bei großen Druckmengen durch seine Wirtschaftlichkeit aus. So können mit dem Rotationsdruckverfahren sehr hohe Bahngeschwindigkeiten und so auch ein großer Durchsatz realisiert werden. Das Rotationsdruckverfahren erfolgt vorzugsweise durch eine Tiefdrucktechnik, wobei sowohl ein direkter als auch ein indirekter Tiefdruck durchgeführt werden kann. Bei einem direkten Tiefdruck wird die Farbe aus Vertiefungen des Druckzylinders, den Näpfchen, direkt auf das Substrat übertragen. Beim indirekten Tiefdruck wird die Farbe aus den Näpfchen zunächst auf einen typischerweise aus Gummi gefertigten Presseur und von dort nachfolgend auf das Nonwoven aufgebracht. Während mit dem indirekten Tiefdruck ein gleichmäßiger Farbauftrag erreicht werden kann, ermöglicht ein direkter Tiefdruck ohne weiteres auch den Auftrag größerer Farbmengen.

Gemäß einer bevorzugten Ausgestaltung der Erfindung ist vorgesehen, dass der Vliesstoff der Deckschicht aus ungekräuselten Endlosfasern besteht. Gut geeignet für die wirktechnische Einbringung der die Schlaufen bildenden polymeren Fäden ist beispielsweise ein Spinnvlies. Da im Rahmen der Erfindung die Deckschicht auf einen Träger aufkaschiert wird, ist in der Regel ein geringes Flächengewicht der Deckschicht ausreichend. Dabei muss gewährleistet werden, dass bei der Verwendung des Verbundstoffelementes gemeinsam mit einem Hakenband eines Klettverschlusses die von den polymeren Fäden gebildeten Schlaufen nicht ausgerissen werden. Die Festigkeit des Verbundstoffelementes an sich kann jedoch durch eine entsprechend stabile Ausgestaltung des bedruckten Trägers erreicht werden. Grundsätzlich kann aber auch vorgesehen sein, die Deckschicht aus mindestens zwei Vliesstoffschichten zu bilden, in welche die polymeren Fäden eingewirkt sind. Insbesondere können auch Vliesstoffschichten mit unterschiedlichen Faserstrukturen kombiniert werden. Bei einer solchen mehrschichtigen Ausgestaltung der Deckschicht trägt diese wesentlich zu der erforderlichen Festigkeit des gesamten Verbundstoffelementes bei.

Gemäß der vorliegenden Erfindung besteht der Träger aus Nonwoven. Unter Nonwoven werden flächige Faservliese verstanden. Der Begriff umfasst sowohl Stapelvliesware als auch Spinnvliese aus endlosen Filamentfasern. Aufgrund seiner höheren Reißfestigkeit ist ein Spinnvlies oder ein mehrlagiges Nonwoven-Material mit zumindest einer Außenlage aus Spinnvlies in besonderem Maße geeignet. Bevorzugt ist ein mehrlagiges Nonwoven, welches Außenschichten aus Spinnvlies (Spunbond S) und dazwischen zumindest eine Schicht aus schmelzgeblasenen Fasern (Meltblown M) aufweist. Diese mehrlagigen Nonwoven-Materialien mit einer Schichtstruktur SMS, SMMS oder SMMMS zeichnen sich üblicherweise gegenüber einem reinen Spinnvlies durch eine gleichmäßigere Oberflächenstruktur und somit eine bessere Bedruckbarkeit aus. Das Nonwoven kann aus Polyolefinfasern, Polyamidfasern, Polyesterfasern oder Fasermischungen der genannten Materialien bestehen. Das Flächengewicht des als Träger eingesetzten Nonwovens liegt zweckmäßig in einem Bereich zwischen 10 g/m² und 30 g/m². Der eingesetzte Vliesstoff ist vorzugsweise ein SMS aus Polypropylen im Gewichtsbereich von 15 g/m².

Die in den Vliesstoff der Deckschicht eingewirkten polymeren Fäden können ohne Einschränkung als Mono- oder Multifilamente ausgeführt sein. Bevorzugt werden nicht texturierte Fäden, insbesondere nicht texturierte Multifilamente, eingesetzt, da diese kostengünstig und leicht zu verarbeiten sind. Darüber hinausgehend können die von einem solchen Faden gebildeten Schlaufen bei der Verwendung des Verbundstoffelementes als Teil eines Klettverschlusses leicht mit zugeordneten Hakenelementen verbunden werden. Der Erfindung liegt dabei die Erkenntnis zugrunde, dass zur Erzeugung einer festen Verbindung die Hakenelemente eines Klettverschlusses an den gebildeten Schlaufen die polymeren Fäden vorzugsweise vollständig hintergreifen. Für bestimmte Anwendungsfälle kann es jedoch auch zweckmäßig sein, eine Texturierung der Fäden vorzusehen.

Die polymeren Fäden werden zweckmäßigerweise durch ein Nähwirkverfahren in den Vliesstoff der Deckschicht eingebracht. Geeignet ist insbesondere die Nähwirktechnologie Maliwatt, bei der ein Schiebernadel-Schließdrahtsystem den Vliesstoff durchsticht und bei einer Rückwärtsbewegung typischerweise ein oder zwei eingelegte Nähfäden durch den Vliesstoff zieht. Grundsätzlich können jedoch auch andere Nähwirkverfahren wie beispielsweise das Raschelverfahren zur Einwirkung der polymeren Fäden eingesetzt werden. Die Anzahl der Kettfäden beträgt dabei üblicherweise 3 bis 25 pro 2,45 cm, vorzugsweise etwa 20 pro 2,54 cm. Durch einen solchen Abstand der Kettfäden wird eine ausreichende Schlingenzahl für das Verhaken mit einem Hakenband gebildet. Die Maschenzahl beträgt vorzugsweise 2 bis 4 pro cm.

Das Bedrucken von nicht gewebten Textilmaterialien ist bekannt und bei Zellulosesubstraten unproblematisch. Schwieriger ist das Bedrucken von Nonwoven aus Polyamidfasern, Polyesterfasern und vor allem Polyolefinfasern. Insbesondere auf faserartigen Polyolefinstrukturen weisen konventionell benutzte Druckfarben und Farbstoffe nur eine begrenzte Adhäsion auf, was sich nachteilig auf die Wisch- und Abriebsfestigkeit eines Druckbildes auswirkt. Eine geringere Wisch- und Abriebsfestigkeit kann bei dem erfindungsgemäßen Verbundstoffelement in Kauf genommen werden, da die oberseitige textile Deckschicht, die durch Wirken hergestellte freie Schlaufen für den Eingriff von Hakenelementen aufweist und selbst nicht bedruckt ist, das Druckbild schützt. Das Druckbild ist auf der zur Deckschicht zugewandten Seite angeordnet und wird unterseitig von dem Nonwoven und oberseitig von der textilen Deckschicht geschützt.

Um die Bedruckbarkeit und die Qualität des Druckbildes zu verbessern, wird die zu bedruckende Oberfläche des Nonwovens erfindungsgemäß vorbehandelt. Zusätzlich soll bei dem Rotationsdruckverfahren ein Durchschlagen der Druckfarbe durch das Nonwoven vermieden werden, da ansonsten die Walzen der Druckmaschine verschmutzen können. Abgesehen davon, dass derartige Verschmutzungen eine aufwendige Reinigung erfordern, können antrocknende Farbreste auch zu einem Zerfasern oder sogar zu einer Zerstörung des Nonwoven beitragen, so dass insbesondere auch zur Vermeidung eines Durchschlagens der Druckfarbe eine Vorbehandlung der zu bedruckenden Oberfläche des Nonwovens zweckmäßig ist. Schließlich ist auch ein Durchschlagen eines Kaschierklebers bei der Verbindung des Nonwovens mit der Deckschicht zu vermeiden. Es bieten sich verschiedene Möglichkeiten einer Vorbehandlung an. Eine erste Ausgestaltung der Erfindung sieht eine Vorbehandlung der Oberfläche des Nonwovens mit einem thixotropen Primer vor. Der Primer ist eine Schicht, welche die Druckfarbe fixiert und in einem geeigneten Bindemittel anorganische Füllstoffe, z. B. Siliziumdioxid, Titandioxid, Calciumcarbonate, calcinierten Ton oder dergleichen zur Verbesserung der Absorption und Haftung der Druckfarbe enthält. Der thixotrop eingestellte Primer verhält sich unter Scherspannung wie eine Flüssigkeit und lässt sich aufgrund seiner thixotropen Eigenschaften als dünner Film auf die Faseroberfläche des Nonwovens auftragen. Ohne Scherbeanspruchung hat der Primer die Eigenschaften eines Feststoffes, der an der Faseroberfläche haftet. Die thixotropen Eigenschaften erleichtern das Aufbringen des Primers. Für den Primer können die für Druckfarben üblichen Bindemittel wie beispielsweise Nitrocellulose (NC), Polyvinylbutyral (PVB) oder Polyvinylchlorid (PVC) eingesetzt werden. Es versteht sich, dass der Primer als Bindemittel auch Harze enthalten kann, die beispielsweise mit einem bei niedrigen Temperaturen aushärtenden Reaktionsmittel vernetzen. Der Primer kann in einer Menge zwischen 0,1 g/m² und 20 g/m², vorzugsweise zwischen 0,5 g/m² und 2 g/m², aufgetragen werden.

Gemäß einer weiteren Ausführungsvariante der Erfindung weist die zu bedruckende Oberfläche des Nonwovens eine durch Sprühen aufgebrachte Schicht auf, die eine im Wesentlichen geschlossene Haut bildet und eine Schichtdicke von weniger als 5 µm aufweist. Auf der geschlossenen Oberfläche der aufgesprühten Schicht kann ein qualitativ hochwertiges Druckbild erzeugt werden, da die Schicht ein Durchschlagen der Druckfarbe verhindert.

Auf der zu bedruckenden Oberfläche des Nonwovens kann ferner gemäß einer weiteren Ausführungsvariante der Erfindung eine feinporöse Schicht zur Absorption der Druckfarbe aufgebracht sein. Feinporöse Beschichtungen sind als so genannte "Foamcoatings" bekannt und werden beispielsweise zur Appretur von Baumwolltextilien eingesetzt. Die erfindungsgemäße Vorbehandlung der Nonwovenschichten mit einer feinporösen Schicht verbessert die Bedruckbarkeit der nicht gewebten Schichten, insbesondere wenn diese aus polyolefinischen Fasern bestehen. Es kann auf entsprechend vorbehandelten Nonwovenschichten ein besseres Druckbild erzeugt werden, wobei gleichzeitig die Abriebsfestigkeit und Wischfestigkeit verbessert wird.

Zusätzlich zu den beschriebenen Möglichkeiten der Vorbehandlung ist das Nonwoven an seiner bedruckten Seite vorzugsweise durch eine Corona-Entladung vorbehandelt. Durch eine solche Behandlung kann die Oberflächenstruktur der Fasern des Nonwoven derart modifiziert werden, dass diese mit einer zusätzlichen Beschichtung versehen werden können. Das Nonwoven kann auch mit einem Plasmaprozess, insbesondere einem Plasmabeschichtungsprozess, vorbehandelt werden: Geeignet sind insbesondere Plasmapolymerisationsprozesse, bei denen die Oberfläche durch eine Abscheidung bestimmter Materialien aus dem Plasma modifiziert werden. Aus prozesstechnischen Gründen werden vorzugsweise Plasmaverfahren eingesetzt, die bei Atmosphärendruck durchgeführt werden können, da dabei eine aufwendige Vakuumanordnung, durch die das Nonwoven geführt wird, nicht erforderlich ist.

Im Rahmen der Erfindung können die Deckschicht und der aus Nonwoven bestehende Träger vollflächig oder auch abschnittsweise, beispielsweise in einem Muster miteinander verklebt sein.

Ein weiterer Aspekt der vorliegenden Erfindung betrifft bei einem gattungsgemäßen Verbundstoffelement, welches dadurch gekennzeichnet ist, dass der Träger aus Nonwoven besteht, die Verklebung der Deckschicht mit dem Träger. Dabei ist vorgesehen, dass der Klebstoff in einem Muster auf dem Träger appliziert ist, wobei das Muster einen Klebstoffrahmen mit vollflächigem Klebstoffauftrag und innerhalb des Klebstoffrahmens eine Klebestruktur aus regelmäßig angeordneten Klebeflächen und klebstofffreien Bereichen aufweist und wobei der Klebstoffrahmen den Rand des Verbundstoffelementes bildet. Das Verkleben bzw. die Kaschierung kann mit einem reaktiven PUR-Klebstoff oder einem Schmelzkleber erfolgen. Da sowohl der Träger als auch die Deckschicht aus textilem Material gebildet sind, weist das Verbundstoffelement eine gute Luftdurchlässigkeit auf. Durch die lediglich teilflächige Verklebung wird das Verhaken des Verbundstoffelementes mit Kletthaken erleichtert, da diese die männlichen Teile eines Klettverschluss bildenden Elemente tief in die Deckschicht eingreifen können. Das vollflächige Verkleben des Randbereichs des erfindungsgemäßen Verbundstoffelementes verhindert ein randseitiges Aus- oder Abreißen der Deckschicht von dem Träger bei einer Zugbelastung, beispielsweise bei einer Öffnung eines unter Verwendung des Verbundstoffelementes gebildeten Klettverschlusses. Insbesondere kann ein unter Verwendung des Verbundstoffelementes gebildeter Klettverschluss ohne Funktionsbeeinträchtigungen mehrfach benutzt werden. Außerdem können sich die Kletthaken in den verklebten Randbereichen des Verbundstoffelementes weniger intensiv verhaken, wodurch das Material bei einem Öffnen dort lokal einer geringeren Beanspruchung ausgesetzt ist.

Die Klebeflächen bilden innerhalb des Klebstoffrahmens vorzugsweise eine streifen-, gitter-, punkt- oder zellenförmige Struktur, wobei der Anteil der Klebeflächen innerhalb des Klebstoffrahmens zweckmäßigerweise zwischen 10 % und 70 %, bevorzugt zwischen 40 % bis 60 % bezogen auf die von dem Klebstoffrahmen eingeschlossene Fläche beträgt. Der Anteil der verklebten Fläche sowie Art und Form des Klebstoffauftrags richten sich nach den typischen Öffnungskräften bei der Verwendung des Verbundstoffelementes in Kombination mit einem geeigneten Klettband.

Um bei der beschriebenen Ausgestaltung eine rapportgenaue Ablängung und Applikation der einzelnen Verbundstoffelemente zu ermöglichen, können Rapportmarken vorgesehen sein, die auf den Verbundstoff aufgedruckt sind. Die Rapportmarken können dabei sichtbar in das Dekormotiv gedruckt werden. Ebenso kann eine unsichtbare Rapportmarke vorgesehen sein, die beispielsweise mit einer nur unter UV-Licht sichtbaren Farbe gedruckt ist. Grundsätzlich kann auch der Dekoraufdruck erfasst und für eine rapportgenaue Handhabung der Verbundstoffelemente ausgewertet werden.

## Patentansprüche

1. Verbundstoffelement für einen Klettverschluss, insbesondere einen Windelverschluss, mit einem Träger und einer auf dem Träger aufkaschierten textilen Deckschicht, die freie Schlaufen für den Eingriff von Hakenelementen aufweist, wobei die Deckschicht aus einem Vliesstoff und darin eingewirkten polymeren Fäden, welche die Schlaufen bilden, besteht, wobei die Schlaufen lediglich in den Vliesstoff der Deckschicht eingewirkt sind und wobei die Deckschicht mit dem Träger verklebt ist, **dadurch gekennzeichnet, dass** der Träger aus Nonwoven besteht und auf der zur Deckschicht zugewandten innenliegenden Seite bedruckt ist, wobei die Druckfarbe aufgebracht ist:
(i) auf einer mit einem thixotropen Primer vorbehandelten Oberfläche des Nonwovens oder
(ii) auf einer durch Sprühen auf das Nonwoven aufgetragenen Schicht, die eine im Wesentlichen geschlossene Haut auf dem Nonwoven bildet und eine Schichtdicke von weniger als 5 µm aufweist, oder
iii) auf einer auf der zu bedruckenden Oberfläche des Nonwovens aufgebrachten feinporösen Schicht zur Adsorption der Druckfarbe.

2. Verbundstoffelement nach Anspruch 1, **dadurch gekennzeichnet, dass** der Vliesstoff der Deckschicht aus ungekräuselten Endlosfasern besteht.

3. Verbundstoffelement nach Anspruch 1 oder 2, **dadurch gekennzeichnet, dass** die Deckschicht aus mindestens zwei Vliesstoffschichten mit unterschiedlichen Faserstrukturen gebildet ist.

4. Verbundstoffelement nach einem der Ansprüche 1 bis 3, **dadurch gekennzeichnet, dass** das Nonwoven der Trägerschicht aus einem Spinnvlies besteht.

5. Verbundstoffelement nach einem der Ansprüche 1 bis 3, **dadurch gekennzeichnet, dass** das Nonwoven der Trägerschicht mehrlagig ist und zumindest eine Außenlage aus Spinnvlies aufweist.

6. Verbundstoffelement nach einem der Ansprüche 1 bis 5, **dadurch gekennzeichnet, dass** das Nonwoven der Trägerschicht ein Flächengewicht von 10 bis 30 g/m² aufweist.

7. Verbundstoffelement nach einem der Ansprüche 1 bis 6, **dadurch gekennzeichnet, dass** das Nonwoven an seiner bedruckten Seite durch eine Coronaentladung oder einem Plasmaprozess, insbesondere einen Plasmabeschichtungsprozess, vorbehandelt ist.

8. Verbundstoffelement nach einem der Ansprüche 1 bis 7, **dadurch gekennzeichnet, dass** die Deckschicht mit dem Nonwoven des Träger vollflächig miteinander verklebt sind.

9. Verbundstoffelement nach einem der Ansprüche 1 bis 7, **dadurch gekennzeichnet, dass** der Klebstoff zwischen der Deckschicht und dem Träger in einem Muster appliziert ist, das einen Klebstoffrahmen mit vollflächigem Klebstoffauftrag und innerhalb des Klebstoffrahmens eine Klebestruktur aus regelmäßig angeordneten Klebeflächen und klebstofffreien Bereichen aufweist, wobei der Klebstoffrahmen den Rand des Verbundstoffelementes bildet.

10. Verbundstoffelement nach Anspruch 9, **dadurch gekennzeichnet, dass** die Klebeflächen innerhalb des Klebstoffrahmens eine streifenförmige, gitterförmige, punktförmige oder dellenförmige Struktur bilden.

11. Verbundstoffelement nach Anspruch 9 oder 10, **dadurch gekennzeichnet, dass** der Anteil der Klebeflächen innerhalb des Klebstoffrahmens 10 bis 70 %, vorzugsweise 40 bis 60 %, bezogen auf die von dem Klebstoffrahmen eingeschlossene Fläche beträgt.

## Claims

1. Composite material element for a hook and loop fastener, particularly a nappy closure, with a support and a textile covering layer laminated on the support, which has free loops for engagement of hook elements, wherein the cover layer is made from a nonwoven fabric and polymer threads knitted therein, forming the loops, wherein the loops are only knitted into the nonwoven fabric of the cover layer and wherein the cover layer is joined adhesively to the carrier, **characterised in that** the support consists of nonwoven and is positioned on the inner side facing towards the cover layer, wherein the printing ink is applied:
(i) to a surface of the nonwoven that has been treated with a thixotropic primer or
(ii) to a layer that has been sprayed onto the nonwoven, forming a substantially closed skin on the nonwoven and having a layer thickness less than 5 µ, or
(iii) to a fine-pored layer applied to the surface of the nonwoven to which printing is to be added, in order to adsorb the printing ink.

2. Composite material element according to claim 1, **characterised in that** the nonwoven material of the cover layer consists of uncrimped continuous filament fibres.

3. Composite material element according to claim 1 or 2, **characterised in that** the cover layer is formed by at least two nonwoven layers having different fibre structures.

4. Composite material element according to any one of claims 1 to 3, **characterised in that** the nonwoven in the support layer consists of a spun-bonded fabric.

5. Composite material element according to any one of claims 1 to 3, **characterised in that** the nonwoven in the support layer consists of multiple plies and has at least one outer ply made from a spun-bonded fabric.

6. Composite material element according to any one of claims 1 to 5, **characterised in that** the nonwoven in the support layer has a grammage from 10 to 30 g/m².

7. Composite material element according to any one of claims 1 to 6, **characterised in that** the printed side of the nonwoven is pretreated with a corona discharge or plasma process, particularly a plasma coating process.

8. Composite material element according to any one of claims 1 to 7, **characterised in that** the cover layer and the nonwoven in the support are bonded to each other adhesively over the full area thereof.

9. Composite material element according to any one of claims 1 to 7, **characterised in that** the adhesive between the cover layer and the support is applied in a pattern that includes an adhesive frame with application of the adhesive over the full area as well as regularly arranged adhesive areas and areas without adhesive, wherein the with adhesive frame forms the borer of the composite material element.

10. Composite material element according to claim 9, **characterised in that** adhesive create a strip-like, lattice-like punctiform or indented structure within the adhesive frame.

11. Composite material element according to claim 9 or 10, **characterised in that** the adhesive surfaces within the adhesive frame constitute a proportion of 10 to 70 %, preferably 40 to 60 % relative to the surface area enclosed by the adhesive frame.

## Revendications

1. Élément en matière composite pour une fermeture auto-agrippante, avec un support et une couche de recouvrement textile contrecollée sur le support qui comporte des boucles libres pour l'engagement d'éléments en crochets, la couche de recouvrement étant conçue en une nappe de fibres et en des fils polymères tricotés dans celui-ci, qui forment les boucles, les boucles étant tricotés uniquement dans la nappe de fibres de la couche de recouvrement et la couche de recouvrement étant collée avec le support, **caractérisé en ce que** le support est constitué de non-tissé et est imprimé sur la face intérieure dirigée vers la couche de recouvrement, l'encre d'imprimerie étant appliquée :
(i) sur une surface du non-tissé prétraitée avec une couche de fond thixotrope ou
(ii) sur une couche appliquée par vaporisation sur le non-tissé qui forme une pellicule sensiblement fermée sur le non-tissé et qui présente une épaisseur de couche inférieure à 5 µm ou
(iii) sur une couche à pores fins appliquée sur la surface à imprimer, pour l'adsorption de l'encre d'imprimerie.

2. Élément en matière composite selon la revendication 1, **caractérisé en ce que** la nappe de fibres de la couche de recouvrement est constituée de fibres sans fin non crêpées.

3. Élément en matière composite selon la revendication 1 ou la revendication 2, **caractérisé en ce que** la couche de recouvrement est constituée d'au moins deux couches de nappe de fibres avec différentes structures de fibres.

4. Élément en matière composite selon l'une quelconque des revendications 1 à 3, **caractérisé en ce que** le non-tissé de la couche de support est constitué d'un filé-lié.

5. Élément en matière composite selon l'une quelconque des revendications 1 à 3, **caractérisé en ce que** le non-tissé de la couche de support est multicouches et comporte au moins une couche extérieure en filé-lié.

6. Élément en matière composite selon l'une quelconque des revendications 1 à 5, **caractérisé en ce que** le non-tissé de la couche de support présente un grammage de 10 à 30 g/m².

7. Élément en matière composite selon l'une quelconque des revendications 1 à 6, **caractérisé en ce que** sur sa couche imprimée, le non-tissé est prétraité par une décharge Corona ou un processus au plasma, notamment un processus de revêtement au plasma.

8. Élément en matière composite selon l'une quelconque des revendications 1 à 7, **caractérisé en ce que** la couche de recouvrement et le non-tissé du support sont collés l'un sur l'autre à pleine surface.

9. Élément en matière composite selon l'une quelconque des revendications 1 à 7, **caractérisé en ce que** l'agent adhésif entre la couche de recouvrement et le support est appliqué selon un motif qui comporte un cadre d'agent adhésif avec une application de matière adhésive à pleine surface et à l'intérieur du cadre d'agent adhésif, une structure de collage en surfaces de collages disposées régulièrement et en zones exemptes d'agent adhésif, le cadre d'agent adhésif formant le bord de l'élément en matière composite.

10. Élément en matière composite selon la revendication 9, **caractérisé en ce que** les surfaces de collage à l'intérieur du cadre d'agent adhésif forment une structure en forme de bandes, en forme de grille, en forme de points ou en forme de capitons.

11. Élément en matière composite selon la revendication 9 ou la revendication 10, **caractérisé en ce que** le taux des surfaces de collage à l'intérieur du cadre d'agent adhésif s'élève à de 10 à 70 %, de préférence à de 40 à 60 % en rapport à la surface entourée par le cadre d'agent adhésif.
